# EUROPÄISCHE PATENTSCHRIFT

(11) **EP 0 793 949 B1**
(45) Veröffentlichungstag und Bekanntmachung des Hinweises auf die Patenterteilung: **05.06.2002**
(21) Anmeldenummer: 96118745.7
(22) Anmeldetag: 22.11.1996
(51) Int. Cl.: A61F 2/66, A61F 2/76

(54) **Gelenkloser Kunstfuss**
Jointless artificial foot
Pied artificiel sans articulation

(30) Priorität: 04.03.1996 FR 9602990
(43) Veröffentlichungstag der Anmeldung: 10.09.1997
(73) Patentinhaber: Otto Bock HealthCare GmbH, 37115 Duderstadt (DE)
(72) Erfinder: Martin, Pierre, 26270 Loriol (FR); Chabloz, Pierre, 38450 Vif (FR)
(74) Vertreter: Gramm, Werner, Prof. Dipl.-Ing.

(56) Entgegenhaltungen:
- EP-A- 0 444 883
- EP-A- 0 648 479
- WO-A-94/10943
- DE-A- 3 741 487
- DE-A- 4 037 928
- DE-A- 4 038 063
- DE-C- 807 214
- FR-A- 583 917
- FR-A- 2 626 463
- FR-A- 2 640 499
- FR-A- 2 698 538
- GB-A- 2 286 125
- US-A- 3 335 428
- US-A- 4 959 073
- US-A- 5 376 139

## Beschreibung

Erste Lösungsvorschläge sahen für den Kunstfuß eine starre, z.B. aus Holz bestehende Ausführung vor, die später mit einem Gelenk versehen wurde, um die Funktion des Knöchels nachzuahmen. Bei einer Weiterentwicklung wurde dann bereits ein federelastischer, aus Blattfedern zusammengesetzter Fußeinsatz vorgesehen, der mit Schaumstoff umkleidet wurde (siehe z.B. US-A-4,959,073).

Die DE 40 38 063 C2 offenbart einen gelenklosen Prothesenfuß mit einem einteilig ausgebildeten, zumindest eine Plantar- und Dorsalflexion sowie eine axiale Kompression ermöglichenden Fußeinsatz, der im Fußlängsschnitt eine angenähert S-förmige Ausbildung aufweist. Der obere Schenkel bildet mit einem sich unter einem stumpfen Winkel anschließenden vorderen Schrägschenkel ein insgesamt starres Winkelelement, an dessen unteres Ende sich ein mittlerer, blattfederähnlicher Schenkel anschließt, der an seinem hinteren Ende über eine angenähert halbkreisförmige Schenkelverbindung mit dem unteren Schenkel verbunden ist. Dabei erstreckt sich das untere Ende des starren Winkelelementes nach vorn bis etwa in den Bereich der Zehen-Grundgelenke.

Die FR-A1-26 40 499 offenbart einen gelenklosen Kunstfuß mit einem innerhalb eines Fußformteils angeordneten federelastischen Fußeinsatz, der im Längsschnitt angenähert C-förmig mit nach hinten liegender Öffnung ausgebildet ist und die Prothesenbelastung mit seinem oberen C-Schenkel aufnimmt und über seinen unteren C-Schenkel auf eine mit ihm verbundene Blattfeder überträgt, die sich angenähert parallel zum Sohlenbereich nach vorn über den Fußeinsatz hinaus erstreckt und mit ihrem vorderen Ende bis in den Fußspitzenbereich ragt. Der Mittelteil dieses Fußeinsatzes liegt etwa im vorderen Längendrittel der Sohle, während der obere C-Schenkel eine Verbindung mit der Beinprothese bildet. Der C-förmige Fußeinsatz übernimmt eine Federfunktion, die durch ein zwischen die beiden C-Schenkel gelegtes federelastisches Polster ergänzt wird, um eine gewisse Geschmeidigkeit beim Aufsetzen des Fußes zu erreichen. Die sich über angenähert die volle Länge des Kunstfußes erstrekkende Blattfeder ist auf ihrer Unterseite über etwa 2/3 ihrer Länge überwiegend konkav und über ihr vorderes Drittel überwiegend konvex ausgebildet. Jedoch hat sich in der Praxis gezeigt, dass auch dieser Kunstfuß keinen natürlichen Gangablauf ermöglicht.

Einen weiteren gelenklosen Kunstfuß offenbart das deutsche Gebrauchsmuster G 93 15 665.0 bzw. die EP-A-0 648 479. Hier ist ein Schaumkunststoff-Fußformteil mit einem metallischen Versteifungskörper vorgesehen, der durch ein liegendes, nach hinten offenes U-Profil gebildet wird, dessen jeweilige Schenkel bei Belastung elastisch aufeinander zu bewegbar sind. Die auslaufenden Schenkelabschnitte weisen zu ihrem freien Ende hin eine abnehmende Materialstärke auf, während die Schenkelenden mit Verdickungen versehen sind. Der untere U-Schenkel ist an seinem freien Ende mit einer Blattfeder verschraubt, während der obere U-Schenkel mit der Aufnahme eines Beinanschlußteils verbunden ist. Die Blattfeder kann aus Kohlefaser oder Titan bestehen, wobei die Unterseite der Blattfeder in dem nach vorn über den Fußeinsatz hinaus reichenden Abschnitt überwiegend konvex ausgebildet ist und im Ballenbereich eine nach unten gerichtete Kröpfung aufweist, die dem Abrollprofil des Fußes entsprechend geformt ist. Das vordere Ende der Blattfeder ist in einem Vorfußkern, ihr hinteres Ende in einer im Fersenbereich liegenden Lasche eingebettet. Die mit den Verdickungen versehenen Schenkelenden der C-Schenkel weisen übereinander liegende Bohrungen auf, durch die von unten ein Bolzen durch die beiden Schenkelenden steckbar und mit einer Aufnahme eines Beinanschlussteils verschraubbar ist. Der Zwischenraum zwischen den freien U-Schenkeln kann mit einem weichen Polyurethanschaum ausgefüllt sein. Dieses Sprungfederelement soll eine Fußbewegung im Sinne einer Pro-Subination um die Fußlängsachse sowie einen natürlichen Bewegungsablauf gestatten.

Der Erfindung liegt die Aufgabe zugrunde, einen gelenklosen Kunstfuß, wie er sich beispielsweise der EP-A-0 648 479 entnehmen lässt, hinsichtlich der Dämpfung des Auftritts der Ferse, der Durchfederung, des Abrollens und der Seitenstabilität zu verbessern, um so dem Träger einen natürlichen Gang zu ermöglichen, wobei der Träger in die Lage versetzt werden soll, sowohl normal zu gehen, als auch körperliche Übungen und Sport zu betreiben.

Diese Aufgabe wird gemäß der Erfindung durch einen gelenklosen Kunstfuß mit den Merkmalen des Anspruchs 1 gelöst.

Dabei ist es zweckmäßig, wenn der Sattel der Blattfeder zu deren hinterem Ende hin kreissegmentförmig hochgezogen ist, wobei der Fußeinsatz auf diesem Sattel vorzugsweise lösbar befestigt ist. Diese Befestigung kann über einen Schraubbolzen oder eine Klemmverbindung erfolgen. Wesentlich ist dabei, dass diese Verbindung mit lichtem Abstand hinter der durch die Zylinderachse geführten Vertikalen liegt. Vor der Befestigungsstelle wird dadurch ein sich keilförmig auf die Befestigungsstelle hin verjüngender Abstand zwischen dem vorderen Abschnitt des unteren C-Schenkels und der darunterliegenden Blattfeder geschaffen. Der so geschaffene Einfederbereich trägt wesentlich zur Erzielung eines natürlichen Bewegungsablaufs bei.

Weitere Merkmale der Erfindung sind Gegenstand der Unteransprüche und werden in Verbindung mit weiteren Vorteilen der Erfindung anhand von Ausführungsbeispielen näher erläutert.
- **Figur 1**: zeigt einen gelenklosen Kunstfuß im Längsschnitt in der Saggitalebene der Prothese;
- **Figur 2**: zeigt stark verkleinert und in schematischer Darstellung einen Schnitt gemäß der Linie II-II in Figur 1;
- **Figur 3**: zeigt in verkleinertem Maßstab den in Figur 1 dargestellten Fußeinsatz in Draufsicht;
- **Figur 4**: zeigt für den Fußeinsatz eine abgewandelte Ausführungsform in einer Darstellung gemäß Figur 3 und
- **Figur 5**: zeigt für einen gelenklosen Kunstfuß eine Ausführungsvariante in einer Darstellung gemäß Figur 1.

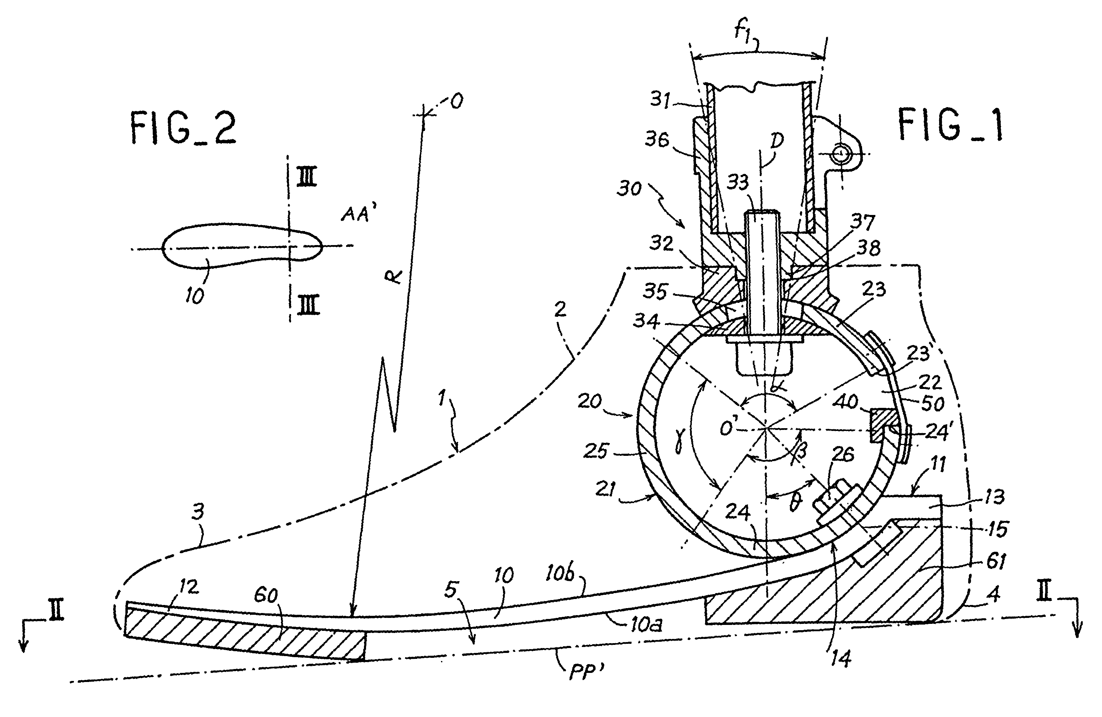

Der in Figur 1 dargestellte gelenklose Kunstfuß weist eine kosmetische Hülle 1 aus geeignetem Material auf, die einen Knöchelbereich 2, einen Fußspitzenbereich 3, einen Fersenbereich 4 und einen Sohlenbereich 5 definiert.

Vorgesehen ist ferner eine Blattfeder 10, die sich angenähert parallel zum Sohlenbereich 5 erstreckt und mit ihrem vorderen Ende 12 bis in den Fußspitzenbereich 3 ragt. Die Blattfeder 10 kann aus unterschiedlichen Materialien bestehen, vorzugsweise aus imprägnierten Glasfasern in einer Matrix aus polymerem Kunstharz. Die Blattfeder 10 weist eine vorzugsweise asymmetrische Form auf und ist der Konfiguration eines linken oder rechten Fußes mit einer Längsachse AA' angepaßt, die in der in Figur 1 dargestellten Sagittalebene liegt. Grundsätzlich ist allerdings auch eine bezogen auf die Achse AA' symmetrische Ausbildung der Blattfeder 10 möglich. Die Unterseite 10a der Blattfeder 10 ist über den größten Teil der Länge der Blattfeder 10 konvex ausgebildet, wobei sich die Krümmung aus dem in Figur 1 eingezeichneten Kurvenradius R mit konstantem Mittelpunkt 0 oder aus einer Kombination eines derartigen Radius mit verschiebbarem Mittelpunkt ergibt.

In den beiden in den Figuren 1 und 2 dargestellten Ausführungsbeispielen ist die Blattfeder 10 mit einem hinteren Ansatz 11 versehen, ausgehend von dem die Stärke der Blattfeder 10 zu deren vorderen Ende 12 hin gleichmäßig abnimmt. Der hintere Ansatz 11 bildet einen Anschlag 13 und geht nach vorn über in einen Sattel 14, der durch die Oberseite 10b der Blattfeder 10 definiert ist und eine Krümmung aufweist, die einen Kreisabschnitt mit einem Mittelpunkt bei 0' darstellen kann. Im Bereich dieses Sattels 14 weist die Blattfeder 10 eine angenähert konstante Stärke auf, deren Abnahme erst vor dem Sattel 14 in Richtung auf das vordere Blattfederende 12 einsetzt.

Zusätzlich zu der Blattfeder 10 weist der Kunstfuß noch einen Fußeinsatz 20 auf, der auf dem Sattel 14 aufliegt und mit diesem lösbar verbunden ist. Der Fußeinsatz 20 besteht im wesentlichen aus einem zylindrischen Segment 21 aus einem Verbundwerkstoff, der Robustheit, Elastizität und geringes spezifisches Gewicht gewährleistet. Der Werkstoff kann aus in einer polymeren Kunstharzmatrix eingebetteten Glasfaserlagen bestehen. Das zylindrische Segment 21 definiert eine Zylinderachse 0', hat eine weitgehend konstante Wandungsstärke und ist rückseitig mit einem verhältnismäßig breiten Axialschlitz 22 versehen, durch den das zylindrische Segment 21 im Querschnitt gesehen etwa die Form eines nach hinten offenen C erhält, das sich aus einem oberen, über den Winkelbereich α erstreckenden oberen C-Schenkel 23, einem unteren, sich über den Winkelbereich β erstreckenden C-Schenkel 24 sowie aus einem die beiden Schenkel 23, 24 miteinander verbindenden, sich über den Winkelbereich γ erstreckenden Steg 25 zusammensetzt.

Gemäß Ausführungsbeispiel erfolgt die Festlegung des zylindrischen Segmentes 21 auf dem Sattel 14 durch einen Schraubbolzen 26, der so angeordnet ist, daß seine Bolzenachse 15 den durch die Zylinderachse 0' definierten Mittelpunkt des zylindrischen Segmentes 21 schneidet. Figur 1 läßt ferner erkennen, daß die durch den Schraubbolzen 26 definierte Verbindung des zylindrischen Segmentes 21 mit dem Sattel 14 der Blattfeder 10 gegenüber der durch 0' verlaufenden Vertikalen D um einen Winkel T nach hinten versetzt angeordnet ist, wobei der Winkel T vorzugsweise zwischen 35° und 45°, insbesondere aber bei 40° liegt. Hierdurch wird deutlich, daß die Verbindungsstelle zwischen dem zylindrischen Segment 21 und der Blattfeder 10 gegenüber der Vertikalen D deutlich nach hinten verlagert ist, wodurch sich diese erfindungsgemäße Lösung wesentlich von allen vorbekannten Ausführungsformen unterscheidet. Dabei kann es zweckmäßig sein, wenn bei flach auf einer angenähert horizontalen Auflagefläche PP' aufliegendem Kunstfuß die gemeinsame Ebene von dem freien Ende 24' des unteren C-Schenkels 24 des zylindrischen Segmentes 21 sowie von dessen Zylinderachse 0' angenähert horizontal liegt. Der obere C-Schenkel 23 ist mit einem Adapter 30 verbunden, der bei der Ausführungsform gemäß Figur 1 eine Rohrverlängerung 31 aufweist, die den Unterschenkel bzw. das Schienbein definiert. Der Adapter 30 stützt sich mit einem Ansatz 32 auf dem äußeren Umfang des oberen C-Schenkels 23 ab und ist diesem gegenüber durch einen Schraubbolzen 33 festlegbar, der durch einen sich gegen die Unterseite des oberen C-Schenkels 23 abstützenden Stützkeil 34, ein sich im oberen C-Schenkel 23 in dessen. Umfangsrichtung erstreckendes Langloch 35 geführt und in die Bodenfläche eines rohrförmigen Stutzens 36 geschraubt ist. Letzterer ragt zu seiner Zentrierung mit einem zylindrischen Ansatz 37 in eine entsprechende Ausnehmung 38 in der Oberseite des Adapteransatzes 32. Nach dem Lösen des Schraubbolzens 33 läßt sich der Adapteransatz 32 innerhalb des Winkelbereichs f₁ gegenüber dem oberen C-Schenkel 23 verschieben und in der gewünschten Position durch Anziehen des Schraubbolzens 33 fixieren. Durch Lösen des Schraubbolzens 33 ergibt sich überdies ein gewisser Spielraum zwischen dem Adapteransatz 32 und dem oberen C-Schenkel 23. Hierdurch wird eine Azimut-Einstellung am Kunstfuß selbst auf der Vertikalen D zwischen dem rohrförmigen Stutzen 36 und dem Adapteransatz 32 ermöglicht. Insgesamt erlaubt diese Ausbildung des Fußadapters 30 eine Spitzfuß- oder Sprungbein-Justierung der Prothese sowie durch die genannte Azimut-Verstellung eine lateral konvexe (O-Stellung) bzw. eine lateral konkave (X-Stellung) Gelenkverstellung. Beim Anziehen des Schraubbolzens 33 werden außerdem gleichzeitig alle wesentlichen Teile des Fußadapters 30 in einer Position oberhalb des oberen C-Schenkels 23 fixiert.

Figur 1 läßt ferner erkennen, daß der freie Rand 24' des unteren C-Schenkels 24 mit einem Stoßdämpfer 40 versehen ist, der mit dem freien Rand 23' des oberen C-Schenkels zusammenwirken soll, wenn das zylindrische Segment 21 einer entsprechenden Belastung unterworfen wird.

Bei dem Ausführungsbeispiel gemäß Figur 1 ist der Axialschlitz 22 des zylindrischen Segmentes 21 durch eine Fessel 50 überbrückt, die die beiden C-Schenkel 23, 24 auf der dem Steg 25 gegenüberliegenden Seite miteinander verbindet. Diese Fessel 50 kann beispielsweise ein Stoffgurt sein und dient in erster Linie dazu, ein übermäßiges Auseinanderspreizen der beiden C-Schenkel 23, 24 zu verhindern.

Bei dem Ausführungsbeispiel gemäß Figur 1 ist die Blattfeder 10 auf ihrer Unterseite 10a mit einer Fußspitzsohle 60 und mit einem Fersenkeil 61 bestückt, beispielsweise verklebt. Dabei ist der Fersenkeil 61 vorzugsweise bündig an das hintere Ende 13 der Blattfeder 10 angepaßt, um hier eine wirksame Befestigung zu erzielen und zugleich eine Abstützung für das hintere Blattfederende 13 zu bilden.

Der vorstehend beschriebene Kunstfuß baut besonders leicht und ermöglicht einen natürlichen Gang, wobei die federelastischen Eigenschaften insbesondere durch die nach hinten verlegte Befestigung des zylindrischen Segmentes 21 auf dem hinteren Abschnitt der Blattfeder 10 positiv beeinflußt werden. Der Druck über den Fersenkeil 61 oder über die Ferse der kosmetischen Hülle 1 führt zu einem Zusammendrücken des zylindrischen Segmentes 21, wodurch sich der Axialschlitz 22 teilweise oder völlig schließt. Es folgt eine Absenkung der Prothesenstruktur um eine Achse, die nahe der physiologischen Knöchelachse liegt; diese Achse ist im Ausführungsbeispiel gemäß Figur 1 als Zylinderachse 0' dargestellt. Hierdurch ergibt sich ein natürliches Auftreten, das einen weichen Gang ermöglicht, wodurch insbesondere die Bewegung im Heruntergehen vereinfacht und ein natürliches Abrollen gewährleistet wird, wenn der Fuß nach dem Aufsetzen der Ferse vollständig auftritt und abrollt. Diese aufeinanderfolgenden Phasen werden durch die Wirkung der Blattfeder 10 harmonisiert, deren große Länge sowie leichte Krümmung in der Abrollrichtung eine elastische Verformung begünstigen und diese von der Fußsohle auf die Auflagefläche verlagern, um so die Gewichtsverlagerung der gehenden Person abzufedern. Sobald die Streckphase beendet ist, beginnt die Abrollphase auf Höhe der Blattfeder 10, die fortschreitend vom Zusammendruck entlastet wird. Diese Entlastung führt zu einer Abgabe der durch das zylindrische Segment 21 gespeicherten Energie und dann zu einer Kombination mit den Reaktionen der Blattfeder, um dann mit einer einzigen elastischen Reaktion der Blattfeder abzuschließen, sowie die Fessel 50 gespannt ist. Die Kombination aufeinanderfolgender elastischer Reaktionen bewirkt einen geschmeidigen und leichten Gang, der dem natürlichen Gang sehr nahe kommt.

Konstruktive Details wie Länge und Dicke der Blattfeder 10, Materialstärke und Durchmesser des zylindrischen Segmentes 21 sowie Größe seines Axialschlitzes 22 wird der Fachmann dem jeweiligen Prothesenträger, insbesondere seinem Gewicht anpassen.

Die elastische Verformbarkeit des zylindrischen Segmentes 21 durch dessen Axialschlitz 22 ermöglicht eine relative Verdrehung des oberen C-Schenkels 23 gegenüber dem Steg 25 bzw. dem unteren C-Schenkel 24, so daß Verdrehbewegungen des Fußes um die Beinachse, z.B. bei der Vorwärtsbewegung, möglich sind.

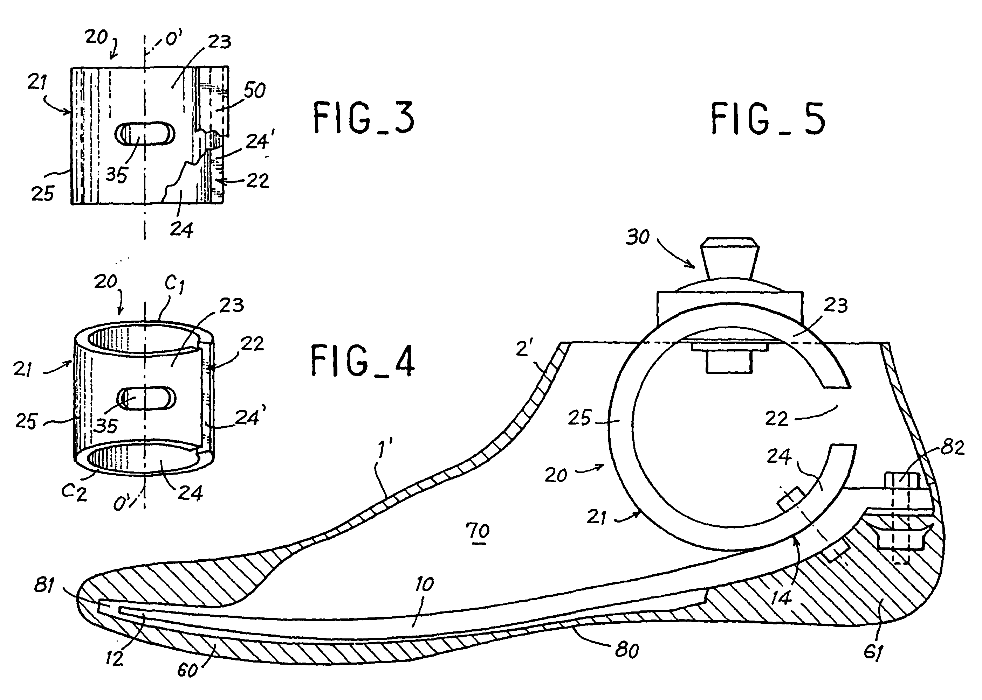

Zur Unterstützung derartiger Bewegungen kann das zylindrische Segment 21 gemäß Figur 4 ausgebildet sein. Hier liegen die beiden Stirnseiten C1, C2 des Fußeinsatzes 20 in Seitenansicht spiegelbildlich schräg zur Zylinderachse 0' und konvergieren radial nach außen, so daß der obere C-Schenkel 23 schmaler ausgebildet ist als der auf dem Sattel 14 montierte untere C-Schenkel 24.

Bei einer abgewandelten Ausführungsform gemäß Figur 5 werden die Fußspitzsohle 60 und der Fersenkeil 61 unmittelbar von einer Hülle 1' gebildet, die einen Hohlraum 70 umschließt, der sowohl die Blattfeder 10 als auch das zylindrische Segment 21 aufnimmt. Letzteres weist gegenüber der Hülle 1' einen lichten Abstand auf, der bei Verformungen des zylindrischen Segmentes 21 und/oder Verdrehungen seines oberen C-Schenkels 23 jede Berührung bzw. Reibung an dem Knöchelbereich 2' unterbindet. Bei dieser Ausführungsform bildet die Hülle 1' auch eine Sohle 80, die die Fußspitzsohle 60 mit dem Fersenkeil 61 verbindet. Figur 5 läßt ferner erkennen, daß das vordere Ende 12 der Blattfeder 10 so in einen vorderen Gleitabschnitt 81 des von der kosmetischen Hülle 1' umschlossenen Hohlraumes 70 ragt, daß zwischen dem Blattfederende 12 und der kosmetischen Hülle 1' noch ein Gleitbereich verbleibt.

Der Fersenkeil 61 der kosmetischen Hülle 1' ist durch ein nur schematisch angedeutetes Befestigungsmittel 82 mit dem hinteren Ende 13 der Blattfeder 10 so verbunden, daß die Hülle 1' gegenüber der Blattfeder 10 keine relative Gleitbewegung entlang der Saggitalebene auszuführen vermag.

Die Erfindung ist nicht auf die in den Figuren dargestellten und vorstehend beschriebenen Ausführungsbeispiele beschränkt; diverse Modifikationen sind möglich, ohne den Bereich der Erfindung zu verlassen.

## Patentansprüche

1. Gelenkloser Kunstfuß für eine Beinprothese mit folgenden Merkmalen:
a) Ein federelastischer Fußeinsatz (20) ist innerhalb eines Fußformteils (1, 2, 3, 4) angeordnet und im Längsschnitt angenähert C-förmig mit nach hinten liegender Öffnung (22) ausgebildet, nimmt die Prothesenbelastung mit seinem oberen C-Schenkel (23) auf und überträgt sie über seinen unteren C-Schenkel (24) auf eine mit ihm verbundene Blattfeder (10);
b) der Fußeinsatz (20) wird im Wesentlichen durch ein liegend angeordnetes rohrförmiges zylindrisches Segment (21) gebildet, das eine horizontale Zylinderachse (O') und zur Bildung der genannten hinteren Öffnung (22) einen Axialschlitz aufweist;
c) die Blattfeder (10) erstreckt sich angenähert parallel zum Sohlenbereich (5) nach vorn über den Fußeinsatz (20) hinaus und ragt mit ihrem vorderen Ende (12) bis in den Fußspitzenbereich (3);
d) die Unterseite (10a) der Blattfeder (10) ist in dem Bereich zwischen Fußeinsatz (20) und dem freien Blattfederende (12) überwiegend konvex ausgebildet;
e) die Oberseite (10b) der Blattfeder (10) bildet in deren hinteren Endbereich einen Sattel (14) zur stützenden Aufnahme und Festlegung eines Abschnittes des unteren C-Schenkel (24) des Fußeinsatzes (20);
f) der obere C-Schenkel (23) ist mit einem Fußadapter (30) zur lösbaren Verbindung mit einer Beinprothese bestückt.

2. Kunstfuß nach Anspruch 1, **dadurch gekennzeichnet, dass** der Sattel (14) der Blattfeder (10) zu deren hinterem Ende (13) hin kreissegmentförmig hochgezogen ist.

3. Kunstfuß nach Anspruch 1 oder 2, **dadurch gekennzeichnet, dass** der Fußeinsatz (20) auf dem Sattel (14) der Blattfeder (10) lösbar befestigt ist.

4. Kunstfuß nach Anspruch 3, **dadurch gekennzeichnet, dass** die Befestigung des unteren C-Schenkels (24) des zylindrischen Segmentes (21) auf dem Sattel (14) der Blattfeder (10) über einen Schraubbolzen (26) oder eine Klemmverbindung erfolgt, der bzw. die mit Abstand (⊖) hinter der durch die Zylinderachse (O') geführten Vertikalen (D) liegt.

5. Kunstfuß nach Anspruch 4, **dadurch gekennzeichnet, dass** bei Verwendung eines Schraubbolzens (26) dessen Bolzenachse (15) mit der durch die Zylinderachse (O') geführten Vertikalen (D) einen Winkel (⊖) von etwa 40° einschließt.

6. Kunstfuß nach einem der vorhergehenden Ansprüche, **dadurch gekennzeichnet, dass** bei flach auf einer angenähert horizontalen Auflagefläche (pp') aufliegendem Kunstfuß die gemeinsame Ebene von dem freien Ende (24') des unteren C-Schenkels (24) des Fußeinsatzes (20) sowie von dessen Zylinderachse (0') angenähert horizontal liegt.

7. Kunstfuß nach einem der vorhergehenden Ansprüche, **dadurch gekennzeichnet, daß** der Fußeinsatz (20) eine konstante Wandungsstärke aufweist.

8. Kunstfuß nach einem der vorhergehenden Ansprüche, **dadurch gekennzeichnet, daß** die Blattfeder (10) Abschnitte unterschiedlicher Dicke und/oder Breite aufweist.

9. Kunstfuß nach einem der vorhergehenden Ansprüche, **dadurch gekennzeichnet, daß** die beiden Stirnseiten (C1, C2) des Fußeinsatzes (20) in Seitenansicht spiegelbildlich schräg zur Zylinderachse (O') liegen und radial nach außen konvergieren, so daß der obere C-Schenkel (23) schmaler ist als der auf dem Sattel (14) montierte untere C-Schenkel (24).

10. Kunstfuß nach einem der Ansprüche 1 bis 8, **dadurch gekennzeichnet, daß** die beiden Stirnseiten (C1, C2) des Fußeinsatzes (20) parallel schräg zur Zylinderachse (O') liegen.

11. Kunstfuß nach einem der vorhergehenden Ansprüche, **dadurch gekennzeichnet, daß** der obere C-Schenkel (23) im Bereich vor dem Fußadapter (30) schmaler ausgebildet ist.

12. Kunstfuß nach einem der vorhergehenden Ansprüche, **gekennzeichnet durch** eine den Axialschlitz (22) des zylindrischen Segmentes (21) überbrückende, die beiden C-Schenkel (23, 24) miteinander verbindende Fessel (50).

13. Kunstfuß nach einem der vorhergehenden Ansprüche, **dadurch gekennzeichnet, daß** im Bereich des Axialschlitzes (22) ein Dämpfer (40) angeordnet ist.

14. Kunstfuß nach einem der vorhergehenden Ansprüche, **dadurch gekennzeichnet, daß** der Fußadapter (30) relativ zum oberen C-Schenkel (23) des Fußeinsatzes (20) in dessen Umfangsrichtung (f₁) verstellbar und in der gewünschten Position am oberen C-Schenkel (23) festlegbar ist.

15. Kunstfuß nach einem der vorhergehenden Ansprüche, **dadurch gekennzeichnet, daß** der Fußadapter (30) gegenüber dem Fußeinsatz (20) in radialer Richtung justierbar ist.

16. Kunstfuß nach einem der vorhergehenden Ansprüche, **dadurch gekennzeichnet, daß** der Fußadapter (30) gegenüber dem Fußeinsatz (20) um die Vertikalachse (D) drehbar und in der gewünschten Position festlegbar ist.

17. Kunstfuß nach einem der vorhergehenden Ansprüche, **dadurch gekennzeichnet, daß** die Befestigung des gegebenenfalls mehrteilig ausgebildeten Fußadapters (30) am Fußeinsatz (20) über einen Schraubbolzen (33) erfolgt, der zum zylindrischen Segment (21) radial ausgerichtet ist und - bezogen auf die Fußlänge - mit Abstand vor der Befestigung (26) des Fußeinsatzes (20) auf der Blattfeder (10) liegt.

18. Kunstfuß nach Anspruch 17, **dadurch gekennzeichnet, daß** der Schraubbolzen (33) durch ein sich in Umfangsrichtung erstreckendes Langloch (35) im oberen C-Schenkel (23) geführt ist.

19. Kunstfuß nach einem der vorhergehenden Ansprüche, **dadurch gekennzeichnet, daß** die Blattfeder (10) auf ihrer Unterseite (10a) mit einem Fersenkeil (61) bestückt ist.

20. Kunstfuß nach Anspruch 19, **dadurch gekennzeichnet, daß** der Fersenkeil (61) an einem hinteren Ansatz (11) der Blattfeder (10) befestigt ist.

21. Kunstfuß nach einem der vorhergehenden Ansprüche, **dadurch gekennzeichnet, daß** die Blattfeder (10) auf ihrer Unterseite (10a) mit einer Fußspitzsohle (60) bestückt ist.

22. Kunstfuß nach Anspruch 19 und 21, **dadurch gekennzeichnet, daß** Fußspitzsohle (60) und Fersenkeil (61) einteilig mit einer diese beiden Abschnitte miteinander verbindenden Sohle (80) ausgebildet sind.

23. Kunstfuß nach einem der vorhergehenden Ansprüche, **gekennzeichnet durch** eine sich bis in den Knöchelbereich (2; 2') erstreckende kosmetische Hülle (1; 1'), die die Blattfeder (10) vollständig und den Fußeinsatz (20) zumindest weitgehend umschließt.

24. Kunstfuß nach Anspruch 23, **dadurch gekennzeichnet, daß** das vordere Ende (12) der Blattfeder (10) so in einen vorderen Gleitabschnitt (81) des von der kosmetischen Hülle (1') umschlossenen Hohlraumes (70) ragt, daß zwischen dem Blattfederende (12) und der kosmetischen Hülle (1') noch ein Gleitbereich verbleibt.

## Claims

1. Jointless artificial foot for a leg prosthesis, with the following features:
a) a resilient foot insert (20) is arranged within a foot moulding (1, 2, 3, 4) and is approximately C-shaped in longitudinal section with an opening (22) situated towards the rear and accommodates the load of the prosthesis with its upper C-arm (23) and transmits said load via its lower C-arm (24) to a leaf spring (10) connected to it;
b) the foot insert (20) is formed substantially by a horizontally arranged tubular cylindrical segment (21) which has a horizontal cylinder axis (0') and an axial slot for the purpose of forming the aforementioned rear opening (22);
c) the leaf spring (10) extends forwards beyond the foot insert (20) approximately parallel to the sole region (5) and projects into the foot-point region (3) with its front end (12);
d) the underside (10a) of the leaf spring (10) is of predominantly convex construction in the region between the foot insert (20) and the free end (12) of the leaf spring;
e) the upper side (10b) of the leaf spring (10) forms in the rear end region thereof a saddle (14) for supportive accommodation and fixation of a section of the lower C-arm (24) of the foot insert (20);
f) the upper C-arm (23) is provided with a foot adapter (30) for detachable connection to a leg prosthesis.

2. Artificial foot according to Claim 1, **characterised in that** the saddle (14) of the leaf spring (10) is raised in the form of a circular segment in the direction towards the rear end (13) of said leaf spring.

3. Artificial foot according to Claim 1 or 2, **characterised in that** the foot insert (20) is detachably fastened to the saddle (14) of the leaf spring (10).

4. Artificial foot according to Claim 3, **characterised in that** the lower C-arm (24) of the cylindrical segment (21) is fastened to the saddle (14) of the leaf spring (10) via a screw bolt (26) or a clamp connection which is situated with a spacing (θ) behind the vertical (D) passing through the cylinder axis (O').

5. Artificial foot according to Claim 4, **characterised by** the use of a screw bolt (26), the axis (15) of which includes an angle (θ) of about 40° with the vertical (D) passing through the cylinder axis (O').

6. Artificial foot according to one of the preceding claims, **characterised in that** in the case of an artificial foot lying flat on an approximately horizontal bearing surface (pp') the common plane pertaining to the free end (24') of the lower C-arm (24) of the foot insert (20) and also pertaining to the cylinder axis (0') thereof is approximately horizontal.

7. Artificial foot according to one of the preceding claims, **characterised in that** the foot insert (20) has a constant wall thickness.

8. Artificial foot according to one of the preceding claims, **characterised in that** the leaf spring (10) comprises sections of differing thickness and/or width.

9. Artificial foot according to one of the preceding claims, **characterised in that** in side view the two end faces (C1, C2) of the foot insert (20) are in mirror-image oblique arrangement in relation to the cylinder axis (O') and converge radially outwards so that the upper C-arm (23) is narrower than the lower C-arm (24) mounted on the saddle (14).

10. Artificial foot according to Claims 1 to 8, **characterised in that** the two end faces (C1, C2) of the foot insert (20) are in parallel oblique arrangement in relation to the cylinder axis (O').

11. Artificial foot according to one of the preceding claims, **characterised in that** the upper C-arm (23) is of narrower construction in the region in front of the foot adapter (30).

12. Artificial foot according to one of the preceding claims, **characterised by** a fetter (50) spanning the axial slot (22) of the cylindrical segment (21) and connecting the two C-arms (23, 24) to one another.

13. Artificial foot according to one of the preceding claims, **characterised in that** a damper (40) is arranged in the region of the axial slot (22).

14. Artificial foot according to one of the preceding claims, **characterised in that** the foot adapter (30) is adjustable relative to the upper C-arm (23) of the foot insert (20) in the peripheral direction (f₁) thereof and is capable of being fixed in the desired position on the upper C-arm (23).

15. Artificial foot according to one of the preceding claims, **characterised in that** the foot adapter (30) is adjustable in the radial direction in relation to the foot insert (20).

16. Artificial foot according to one of the preceding claims, **characterised in that** the foot adapter (30) is rotatable in relation to the foot insert (20) about the vertical axis (D) and is capable of being fixed in the desired position.

17. Artificial foot according to one of the preceding claims, **characterised in that** the optionally multicomponent foot adapter (30) is fastened to the foot insert (20) via a screw bolt (33) which is radially aligned in relation to the cylindrical segment (21) and - with respect to the length of the foot - is situated in front of the fastening (26) of the foot insert (20) to the leaf spring (10) subject to a spacing.

18. Artificial foot according to Claim 17, **characterised in that** the screw bolt (33) is passed through an elongate hole (35) in the upper C-arm (23) extending in the peripheral direction.

19. Artificial foot according to one of the preceding claims, **characterised in that** the leaf spring (10) is provided on its underside (10a) with a heel wedge (61).

20. Artificial foot according to Claim 19, **characterised in that** the heel wedge (61) is fastened to a rear extension (11) of the leaf spring (10).

21. Artificial foot according to one of the preceding claims, **characterised in that** the leaf spring (10) is provided on its underside (10a) with a foot-point sole (60).

22. Artificial foot according to Claims 19 and 21, **characterised in that** foot-point sole (60) and heel wedge (61) are constructed in one piece with a sole (80) connecting these two sections to one another.

23. Artificial foot according to one of the preceding claims, **characterised by** a cosmetic covering (1; 1') extending into the ankle region (2; 2'), said cosmetic covering surrounding the leaf spring (10) completely and surrounding the foot insert (20) at least to a large extent.

24. Artificial foot according to Claim 23, **characterised in that** the front end (12) of the leaf spring (10) projects in such a manner into a front sliding section (81) of the hollow space (70) surrounded by the cosmetic covering (1') that a sliding region still remains between the end (12) of the leaf spring and the cosmetic covering (1').

## Revendications

1. Pied artificiel sans articulation pour une prothèse de la jambe, comprenant les particularités suivantes:
a) Un insert de pied (20) élastique est disposé à l'intérieur d'une pièce en forme de pied (1, 2, 3, 4), et présente en coupe longitudinale sensiblement une forme de "C" ayant son ouverture (22) située vers l'arrière, reçoit la charge de la prothèse par la branche supérieure du "C" (23) et la transmet par la branche inférieure du "C" (24) à un ressort à lame (10) qui lui est raccordé;
b) L'insert de pied (20) est essentiellement formé par un segment cylindrique tubulaire (21) disposé couché, qui présente un axe de cylindre (O') horizontal et une fente axiale formant ladite ouverture arrière (22);
c) Le ressort à lame s'étend vers l'avant au-delà de l'insert de pied (20) sensiblement parallèlement à la zone de plante du pied (5) et fait saillie par son extrémité antérieure (12) jusque dans la zone de pointe du pied (3);
d) La face inférieure (10a) du ressort à lame (10) est de forme principalement convexe dans la zone située entre l'insert de pied (20) et l'extrémité libre (12) du ressort à lame;
e) La face supérieure (10b) du ressort à lame (10) forme dans la zone de son extrémité arrière un siège (14) pour recevoir en appui et positionner une partie de la branche inférieure du "C" (24) de l'insert de pied (20);
f) La branche supérieure du "C" (23) est munie d'un adaptateur de pied (30) pour une liaison amovible avec une prothèse de la jambe.

2. Pied artificiel selon la revendication 1, **caractérisé en ce que** le siège (14) du ressort à lame (10) est relevé en forme de segment de cercle vers l'extrémité arrière (13) du ressort.

3. Pied artificiel selon la revendication 1 ou 2, **caractérisé en ce que** l'insert de pied (20) est fixé de façon amovible sur le siège (14) du ressort à lame (10).

4. Pied artificiel selon la revendication 3, **caractérisé en ce que** la fixation de la branche inférieure du "C" (24) du segment cylindrique (21) sur le siège (14) du ressort à lame (10) s'effectue au moyen d'une vis (26) ou d'une liaison par serrage, qui se trouve à une distance (θ) derrière la verticale (D) passant par l'axe du cylindre (O').

5. Pied artificiel selon la revendication 4, **caractérisé en ce que** dans le cas de l'utilisation d'une vis (26) l'axe (15) de celle-ci définit avec la verticale (D) passant par l'axe du cylindre (O') un angle (θ) d'environ 40°.

6. Pied artificiel selon l'une des revendications précédentes, **caractérisé en ce que** lorsque le pied artificiel est posé à plat sur une surface d'appui (pp') sensiblement horizontale, le plan commun passant par l'extrémité libre (24') de la branche inférieure du "C" (24) de l'insert de pied (20) et par l'axe du cylindre (O') dudit insert de pied est sensiblement horizontal.

7. Pied artificiel selon l'une des revendications précédentes, **caractérisé en ce que** l'insert de pied (20) présente une épaisseur de paroi constante.

8. Pied artificiel selon l'une des revendications précédentes, **caractérisé en ce que** le ressort à lame (10) présente des tronçons d'épaisseur et/ou de largeur différente.

9. Pied artificiel selon l'une des revendications précédentes, **caractérisé en ce qu'**en vue côté, les deux faces d'extrémité (C1, C2) de l'insert de pied (20) s'étendent selon des inclinaisons symétriques par rapport à l'axe du cylindre (O') et convergent radialement vers l'extérieur, de telle sorte que la branche supérieure du "C" (23) est plus étroite que la branche inférieure du "C" (24) montée sur le siège (14).

10. Pied artificiel selon l'une des revendications 1 à 8, **caractérisé en ce que** les deux faces d'extrémité (C1, C2) de l'insert de pied (20) sont inclinées parallèlement par rapport à l'axe du cylindre (O').

11. Pied artificiel selon l'une des revendications précédentes, **caractérisé en ce que** la branche supérieure du "C" (23) est plus étroite dans la zone située en avant de l'adaptateur de pied (30).

12. Pied artificiel selon l'une des revendications précédentes, **caractérisé par** un lien (50) qui forme un pont sur la fente axiale (22) du segment de cylindre (21) et raccorde ensemble les deux branches du "C" (23, 24).

13. Pied artificiel selon l'une des revendications précédentes, **caractérisé en ce qu'**un amortisseur (40) est disposé dans la zone de la fente axiale (22).

14. Pied artificiel selon l'une des revendications précédentes, **caractérisé en ce que** l'adaptateur de pied (30) est réglable par rapport à la branche supérieure du "C" (23) de l'insert de pied (20) dans la direction périphérique (f₁) de celui-ci et peut être fixé dans la position désirée sur la branche supérieure du "C" (23).

15. Pied artificiel selon l'une des revendications précédentes, **caractérisé en ce que** l'adaptateur de pied (30) est ajustable en direction radiale par rapport à l'insert de pied (20).

16. Pied artificiel selon l'une des revendications précédentes, **caractérisé en ce que** l'adaptateur de pied (30) peut tourner autour de l'axe vertical (D) par rapport à l'insert de pied (20) et peut être fixé dans la position désirée.

17. Pied artificiel selon l'une des revendications précédentes, **caractérisé en ce que** la fixation de l'adaptateur de pied (30), formé le cas échéant de plusieurs pièces, sur l'insert de pied (20) s'effectue au moyen d'une vis (33), qui est disposée radialement par rapport au segment de cylindre (21), et se trouve - par rapport à la longueur du pied - à distance en avant de la fixation (26) de l'insert de pied (20) sur le ressort à lame (10).

18. Pied artificiel selon la revendication 17, **caractérisé en ce que** la vis (33) est guidée dans la branche supérieure du "C" (23) par un trou oblong (35) s'étendant dans la direction périphérique.

19. Pied artificiel selon l'une des revendications précédentes, **caractérisé en ce que** le ressort à lame (10) est muni d'une cale de talon (61) sur sa face inférieure (10a).

20. Pied artificiel selon la revendication 19, **caractérisé en ce que** la cale de talon (61) est fixée sur un appendice arrière (11) du ressort à lame (10).

21. Pied artificiel selon l'une des revendications précédentes, **caractérisé en ce que** le ressort à lame (10) est muni sur sa face inférieure (10a) d'une semelle de pointe de pied (60).

22. Pied artificiel selon la revendication 19 et 21, **caractérisé en ce que** la semelle de pointe de pied (60) et la cale de talon (61) sont formées d'une seule pièce avec une semelle (80) reliant ensemble ces deux parties.

23. Pied artificiel selon l'une des revendications précédentes, **caractérisé par** une enveloppe esthétique (1; 1'), s'étendant jusque dans la région de la cheville (2; 2'), qui entoure complètement le ressort à lame (10) et au moins en grande partie l'insert de pied (20).

24. Pied artificiel selon la revendication 23, **caractérisé en ce que** l'extrémité antérieure (12) du ressort à lame (10) s'avance dans une partie de glissement (81) antérieure de la cavité (70) entourée par l'enveloppe esthétique (1'), de telle manière qu'il reste encore une zone de glissement entre l'extrémité (12) du ressort à lame et l'enveloppe esthétique (1').
